Europäisches Patentamt

European Patent Office

Office européen des brevets

(11) Veröffentlichungsnummer: **0 407 989 A1**

# EUROPÄISCHE PATENTANMELDUNG

(21) Anmeldenummer: **90113194.6**

(22) Anmeldetag: **11.07.90**

(51) Int. Cl.5: **C07C 17/10**, C07C 19/08

(30) Priorität: **14.07.89 DE 3923255**

(43) Veröffentlichungstag der Anmeldung:
**16.01.91 Patentblatt 91/03**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Cremer, Hans Robert, Dr.**
**Pappelstrasse 1**
**D-5014 Kerpen(DE)**
Erfinder: **Siegemund, Günter, Dr.**
**Frankfurter Strasse 21**
**D-6238 Hofheim am Taunus(DE)**
Erfinder: **Schnauber, Martin, Dr.**
**Am Forsthaus 1**
**D-6238 Hofheim am Taunus(DE)**

(54) **Verfahren zur Herstellung von 1,1,1-Trifluor-2,2-dichlorethan durch Photochlorierung.**

(57) Die Erfindung betrifft ein Verfahren zur Herstellung von 1,1,1-Trifluor-2,2-dichlorethan, wobei die Photochlorierung in flüssiger Phase durchgeführt wird. Dabei kann die flüssige Phase durch Druck und/oder Kühlung aufrechterhalten werden. Die Reaktionstemperaturen betragen vorzugsweise -80 °C bis +80 °C.

EP 0 407 989 A1

## VERFAHREN ZUR HERSTELLUNG VON 1,1,1-TRIFLUOR-2,2-DICHLORETHAN DURCH PHOTOCHLORIERUNG

Die Erfindung betrifft ein Verfahren zur Herstellung von 1,1,1-Trifluor-2,2-dichlorethan (R 123) durch Photochlorierung von 1,1,1-Trifluor-2-chlorethan (R 133a).

1,1,1-Trifluor-2,2-dichlorethan (R 123) ist eine bekannte Verbindung. Sie gilt als potentieller Ersatzstoff für den vollhalogenierten Fluorchlorkohlenwasserstoff Fluortrichlormethan (R 11), dessen Produktion und Verbrauch wegen des Verdachts einer Schädigung der Ozonschicht eingeschränkt werden muß. R 123 hat aufgrund seines Abbauverhaltens in den unteren Atmosphärenschichten ein erheblich geringeres Ozongefährdungspotential. Es findet Verwendung als Verschäumungsmittel in der Kunststoffverarbeitung und als Reinigungsmittel.

Gemäß US-A-3,755,477 wird 1,1,1-Trifluor-2,2-dichlorethan ($CF_3$-$CHCl_2$) durch Umsetzung von Perchlorethylen mit wasserfreiem Fluorwasserstoff an Chrom-Katalysatoren bei Temperaturen von 360 $^\circ$C erhalten. Nachteilig bei diesem Verfahren sind die rasche Desaktivierung der Katalysatoren und die geringe Ausbeute an $CF_3$-$CHCl_2$ sowie die große Anzahl der wirtschaftlich nicht verwendbaren Nebenprodukte. Zudem ist bei dieser Arbeitsweise nicht auszuschließen, daß sich destillativ nicht abtrennbare, aus toxikologischer Sicht bedenkliche Dichlortrifluorethan-Isomere (z.B. R 123a) bilden.

Weiterhin ist bekannt, daß 1,1,1-Trifluor-2,2-dichlorethan über die thermische Chlorierung von 1,1,1-Trifluorethan zugänglich ist. Wie E.T. McBee et al., Ind. and Engineering Chem., 39, Seite 409-411 (1947) berichten, wird 1,1,1-Trifluorethan bei 497 $^\circ$C mit der 1,1-molaren Chlormenge umgesetzt (Seite 411 und Tabelle III). Dieses Verfahren ist jedoch für eine industrielle Darstellung von R 123 ungeeignet, da weniger als die Hälfte des eingesetzten Chlors abreagiert und R 123 in der Produktpalette nur ein Nebenprodukt darstellt. Unter den angegebenen Bedingungen wird neben dem vollhalogenierten Hauptprodukt $CF_3$-$CCl_3$ auch die Bildung unerwünschter Abbauprodukte (z.B. $CCl_4$, $CF_3Cl$) beobachtet.

Es ist bereits aus der US-A-4,060,469 bekannt, 1,1,1-Trifluor-2,2-dichlorethan durch Photochlorierung von 1,1,1-Trifluor-2-chlorethan in der Gasphase herzustellen. Nachteil dieses Verfahrens ist die sehr geringe Lichtquantenausbeute und der geringe Umsatz. Zudem läßt sich bei dieser Gasphasen-Photochlorierung neben der $CF_3$-$CCl_3$-Bildung auch die Bildung von höhermolekularen Verbindungen ($C_4$- und $C_6$-Verbindungen) beobachten. Nach US-A-4,145,368 und US-A-4,192,822 kann die Ausbeute an R 123 zwar erhöht werden, wenn man die Reaktionsprodukte bei 350 $^\circ$C über Chromoxyfluorid-Katalysatoren leitet. Aber auch dadurch läßt sich die Ausbeute nur auf 14% steigern. Hinzu kommt, daß die verwendeten Cr-O-F-Katalysatoren nicht nur die Dismutierungsreaktion katalysieren, sondern, daß auch Dismutierungs-, Isomerisierungs- und Eliminierungsreaktionen an ihnen stattfinden, und somit in erheblichem Maße wirtschaftlich nicht verwertbare Nebenprodukte bei dieser Arbeitsweise anfallen.

Es bestand daher die Aufgaben, ein Verfahren zur wirtschaftlichen und effizienten Herstellung von 1,1,1-Trifluor-2,2-dichlorethan bereitzustellen.

Gegenstand der Erfindung ist ein Verfahren zur Herstellung von 1,1,1-Trifluor-2,2-dichlorethan durch Photochlorierung von 1,1,1-Trifluor-2-chlorethan, dadurch gekennzeichnet, daß die Photochlorierung in flüssiger Phase durchgeführt wird. Bei dem erfindungsgemäßen Verfahren wird 1,1,1-Trifluor-2,2-dichlorethan in isomerenreiner Form erhalten und als praktisch einziges Nebenprodukt erhält man 1,1,1-Trifluor-2,2,2-trichlorethan.

Wie die flüssige Phase erzeugt wird, ist dabei ohne Bedeutung. Wird sie durch Kühlung unter den Siedepunkt von 1,1,1-Trifluor-2-chlorethan (6,1 $^\circ$C) erzeugt, so kann drucklos chloriert werden.

Chloriert man unter Druck, so kann das Verfahren auch bei höheren Temperaturen durchgeführt werden. Vorzugsweise wird das erfindungsgemäße Verfahren bei Temperaturen von -80 $^\circ$C bis +80 $^\circ$C, insbesondere bei Temperaturen von -30 $^\circ$C bis +25 $^\circ$C ausgeführt, wobei man gleichermaßen gut bei Unter-, Normal- oder Überdruck arbeiten kann.

Weiterhin kann die Chlorierung in einem Lösungsmittel, welches die flüssige Phase darstellt, durchgeführt werden. Dabei kann der Druck und die Temperatur natürlich in einem etwas weiteren Bereich variiert werden als beim lösungsmittelfreien Arbeiten. Vorteilhafterweise werden Lösungsmittel eingesetzt, die oberhalb der Siedetemperatur von R 123 sieden und unter den Chlorierungsbedingungen inert sind. Besonders günstig sind Chlorkohlenwasserstoffe, Fluorkohlenwasserstoffe und Fluorchlorkohlenwasserstoffe, vorzugsweise Tetrachlormethan und Trichlortrifluorethan.

Das Arbeiten in flüssiger Phase gemäß der vorliegenden Erfindung hat gegenüber der bekannten Arbeitsweise in der Gasphase den Vorteil, daß die Lichtquantenausbeute und der Umsatz wesentlich größer sind und auch die Raum-Zeit-Ausbeute erheblich höher ist. Das eingesetzte Chlor reagiert quantitativ ab und braucht daher nicht anschließend aus dem Produktgemisch abgetrennt zu werden.

Bei dem erfindungsgemäßen Verfahren wird im allgemeinen 1,1,1-Trifluor-2-chlorethan mit oder ohne

2

Lösungsmittel in einer mit einem Kühler versehenen Belichtungsapparatur vorgelegt und elementares Chlor unter Belichtung in die Flüssigkeit eingeleitet, wobei sich das Chlor löst und umgehend abreagiert.

Diese Anordnung hat gegenüber der Gasphasenchlorierung den Vorteil, daß die Abtrennung des gebildeten Chlorwasserstoffs wesentlich leichter ist, da der Chlorwasserstoff gasförmig entweicht und destillativ weitgehend abgetrennt werden kann.

Das Molverhältnis Chlor/R 133a sollte im allgemeinen etwa 0,02-1,0 betragen. Wenn hohe Selektivität erwünscht ist, wählt man vorzugsweise ein Molverhältnis von 0,02-0,4. Falls dagegen hoher Umsatz erwünscht ist, wählt man vorzugsweise ein Molverhältnis von 0,4-1,0.

Geeignete Strahlungsquellen sind beispielsweise Metalldampflampen, Edelgasentladungslampen oder Sonnenlicht. Voraussetzung ist nur, daß die Lichtquelle zwischen 2500 Angström und 5200 Angström ein Strahlungsmaximum aufweist.

Das erfindungsgemäße Verfahren läßt sich auch kontinuierlich gestalten, indem am Boden des Belichtungsgefäßes 1,1,1-Trifluor-2-chlorethan und Chlor zugeführt werden und die Produkte durch einen Überlauf kontinuierlich entnommen werden.

In den folgenden Beispielen wird die Erfindung näher erläutert.

Die Produktzusammensetzungen wurden gaschromatographisch analysiert und zusätzlich $^{19}$F- und $^1$H-NMR-spektroskopisch identifiziert. Die angegebenen Prozentangaben sind Gewichtsprozente.

**Beispiel 1:**

In einem mit einer 150 W Quecksilber-Hochdrucklampe und einem Tieftemperaturkühler (-76 °C) versehenen Chlorierungsgefäß (800 ml) wurden unter Kühlung 830 g 1,1,1-Trifluor-2-chlorethan (R 133a) einkondensiert. Unter Belichtung und Rühren wurden 105 g/h Chlor gasförmig in feinverteilter Form am Boden des Gefäßes zugeführt, wobei der Chlorwasserstoff gasförmig über Kopf entwich. Mit Hilfe einer Kühlung wurde die Reaktionstemperatur während des Versuches konstant gehalten. Nach Einleiten der entsprechenden Chlormenge erfolgte die Produktcharakterisierung und die destillative Aufarbeitung.

Die Versuchsresultate bei unterschiedlicher Chlordosierung sind in der nachfolgenden Tabelle aufgeführt.

|  | Molverhältnis Chlor/R 133a | | | | | |
|---|---|---|---|---|---|---|
|  | 0,1 | 0,2 | 0,4 | 0,5 | 0,7 | 0,9 |
| Temperatur (°C) | -10 | -10 | -10 | -10 | -10 | -10 |
| Reaktionszeit(min) | 29 | 57 | 114 | 142 | 199 | 255 |
| Edukte: | | | | | | |
| CF$_3$-CH$_2$Cl (g) | 830 | 830 | 830 | 830 | 830 | 830 |
| Chlor (g) | 50 | 99 | 199 | 248 | 348 | 447 |
| Produkte: | | | | | | |
| CF$_3$-CH$_2$Cl (%) | 87,6 | 76,8 | 59,3 | 52,1 | 38,3 | 28,4 |
| CF$_3$-CHCl$_2$ (%) | 11,4 | 20,1 | 31,4 | 34,9 | 38,7 | 38,7 |
| CF$_3$-CCl$_3$ (%) | 1,0 | 3,1 | 9,2 | 12,9 | 22,9 | 32,8 |
| Umsatz: | | | | | | |
| Chlor (%) | >99 | >99 | >99 | >99 | >99 | >99 |
| R 133a (%) | 9,8 | 18,6 | 33,7 | 40,1 | 53,7 | 64,2 |
| Selektivität: | | | | | | |
| R 123 (%) | 92,0 | 86,6 | 77,1 | 72,9 | 62,7 | 54,0 |

**Beispiel 2:**

In der Versuchsanordnung von Beispiel 1 wurden 600 ml Tetrachlormethan und und 140 g 1,1,1-Trifluor-2-chlorethan vorgelegt und unter Kühlung (+5 °C), Rühren und Belichten 18 g/h Chlor gasförmig eingeleitet. Nach Ablauf der jeweiligen Reaktionszeit erfolgte die destillative Aufarbeitung.

Die Resultate waren wie folgt:

| | Molverhältnis Chlor/R 133a | |
|---|---|---|
| | 0,21 | 0,60 |
| Temperatur (°C) | + 5 | + 5 |
| Reaktionszeit (min) | 60 | 166 |
| Edukte: | | |
| $CF_3$-$CH_2$Cl (g) | 140 | 140 |
| Chlor (g) | 18 | 50 |
| Lösungsmittel: | | |
| $CCl_4$ (ml) | 600 | 600 |
| Produkte: | | |
| (ohne Tetra) | | |
| $CF_3$-$CH_2$Cl (%) | 75,8 | 44,9 |
| $CF_3$-$CHCl_2$ (%) | 21,6 | 38,0 |
| $CF_3$-$CCl_3$ (%) | 2,6 | 17,1 |
| Umsatz: | | |
| Chlor (%) | > 99 | > 99 |
| R 133a (%) | 19,5 | 47,3 |
| Selektivität: | | |
| R 123 (%) | 89,3 | 69,0 |

**Beispiel 3:**

Ein mit einer Kühlung ausgestattetes Belichtungsgefäß (800 ml) wurde mit einer 150 W Quecksilber-Hochdrucklampe und einem Tieftemperaturkühler versehen. Am Boden dieses Gefäßes wurde unter Belichtung und Kühlung (-10 °C) 232 g/h 1,1,1-Trifluor-2-chlorethan (flüssig) und 35 g/h Chlor kontinuierlich zugeführt. Der entstandene Chlorwasserstoff wurde über Kopf abgeleitet und die anderen Produkte durch einen Überlauf kontinuierlich entnommen. Der Versuch wurde 24 Stunden lang durchgeführt. Dabei blieb die Produktzusammensetzung konstant und das Resultat war wie folgt:

Produktzusammensetzung:

$CF_3$-$CH_2$Cl: 72,4 %

$CF_3$-$CHCl_2$: 23,4 %

$CF_3$-$CCl_3$ : 4,2 %

Umsatz:

Chlor : > 99 %

R 133a: 22,0 %

Selektivität:

R 123 : 84,8 %

**Ansprüche**

1. Verfahren zur Herstellung von 1,1,1-Trifluor-2,2-dichlorethan durch Photochlorierung von 1,1,1-Trifluor-2-chlorethan, dadurch gekennzeichnet, daß die Photochlorierung in flüssiger Phase durchgeführt wird.

2. Verfahren nach Anspruch 1, dadurch gekennzeichnet, daß die flüssige Phase durch Druck und/oder Kühlung aufrechterhalten wird.

3. Verfahren nach Anspruch 1, dadurch gekennzeichnet, daß die flüssige Phase durch ein Lösungsmittel gebildet wird.

4. Verfahren nach Anspruch 3, dadurch gekennzeichnet, daß als Lösungsmittel ein Chlorkohlenwasserstoff, ein Fluorkohlenwasserstoff oder ein Fluorchlorkohlenwasserstoff eingesetzt wird.

5. Verfahren nach Anspruch 4, dadurch gekennzeichnet, daß als Lösungsmittel Tetrachlormethan oder 1,1,1-Trichlor-2,2,2,-trifluorethan eingesetzt wird.

6. Verfahren nach einem der Ansprüche 1 bis 5, dadurch gekennzeichnet, daß das Verfahren bei Temperaturen von - 80° C bis + 80° C durchgeführt wird.

7. Verfahren nach einem der Ansprüche 1 bis 5, dadurch gekennzeichnet, daß das Verfahren bei Temperaturen von - 30° C bis + 25° C durchgeführt wird.

8. Verfahren nach einem der Ansprüche 1 bis 7, dadurch gekennzeichnet, daß die für die Photochlorierung verwendete Lichtquelle ein Strahlungsmaximum zwischen 2500 Angström und 5200 Angström aufweist.

9. Verfahren nach einem der Ansprüche 1 bis 8, dadurch gekennzeichnet, daß das Chlor/1,1,1-Trifluor-2-chlorethan-Molverhältnis 0,02 bis 1,0 beträgt.

**Europäisches Patentamt**

# EUROPÄISCHER RECHERCHENBERICHT

Nummer der Anmeldung

**EP 90 11 3194**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | US-A-4 060 469  (SWEENEY)<br>* Ansprüche 1-3,6-13 *<br>— — — | 1-9 | C 07 C 17/10<br>C 07 C 19/08 |
| Y | DE-C-1 002 303  (HOECHST)<br>* Spalte 1, Zeile 23-48; Beispiele 1-3 *<br>— — — | 1-9 | |
| A | DE-A-2 740 585  (HOECHST)<br>* Seite 4, Absatz 3 *<br>— — — | 3,4 | |
| D,A | US-A-4 145 368  (SWEENEY)<br>* Anspruch 1 *<br>— — — — — | 1 | |

| | |
|---|---|
| | **RECHERCHIERTE SACHGEBIETE (Int. Cl.5)**<br><br>C 07 C 17/00<br>C 07 C 19/00 |

Der vorliegende Recherchenbericht wurde für alle Patentansprüche erstellt

| Recherchenort | Abschlußdatum der Recherche | Prüfer |
|---|---|---|
| Berlin | 23 Oktober 90 | PROBERT C.L. |